**Europäisches Patentamt**

**European Patent Office**

**Office européen des brevets**

(19)

(11) Veröffentlichungsnummer : **0 114 283**
**B1**

(12) **EUROPÄISCHE PATENTSCHRIFT**

(45) Veröffentlichungstag der Patentschrift :
01.10.86

(21) Anmeldenummer : 83112351.8

(22) Anmeldetag : 08.12.83

(51) Int. Cl.⁴ : **A 61 B   5/10**

(54) **Gerät für die Brillenoptik zum Messen der Nasenwinkel.**

(30) Priorität : 22.12.82 DE 3247509

(43) Veröffentlichungstag der Anmeldung :
01.08.84 Patentblatt 84/31

(45) Bekanntmachung des Hinweises auf die Patenterteilung : 01.10.86 Patentblatt 86/40

(84) Benannte Vertragsstaaten :
AT CH FR LI

(56) Entgegenhaltungen :
DD-A-   29 846
DD-A-   57 206
FR-A- 2 267 736
FR-A- 2 497 655

(73) Patentinhaber : **Hans Meissburger GmbH**
**Pfinztalstrasse 38**
**D-7500 Karlsruhe 41  (DE)**

(72) Erfinder : **Fischbach, Günther**
**Pfinztalstrasse 38**
**D-7500 Karlsruhe 41  (DE)**

(74) Vertreter : **Dr.-Ing. Hans Lichti Dipl.-Ing. Heiner Lichti**
**Dipl.-Phys. Dr. Jost Lempert**
**Postfach 41 07 60 Durlacher Strasse 31**
**D-7500 Karlsruhe 41  (DE)**

## Beschreibung

Die Erfindung bezieht sich auf ein Gerät für die Brillenoptik zum Messen der Nasenwinkel unter Berücksichtigung der Nasenrückenbreite mit zwei flachen Bauteilen mit je einer schräg verlaufenden Anlagefläche für einen Nasenflügel, wobei die eine Anlagefläche gegenüber der anderen zur Einstellung auf die Nasenrückenbreite linear verstellbar ist und beide Anlageflächen in der Ausgangsposition des Gerätes spiegelsymmetrisch zu einer senkrecht zu der Bauteilebene und zu den Basislinien der Bauteile stehenden Ebene verlaufen.

In der Brillenoptik besteht ein dringendes Bedürfnis zu einer exakten Feststellung der Nasenwinkel, insbesondere des Nasenkeilwinkels unter Berücksichtigung der jeweiligen beim Brillenträger vorhandenen Nasenrückenbreite. Ein derartiges Meßgerät ist bisher in der einschlägigen Technik nicht bekannt geworden. Gemeinhin erfolgt die Feststellung eines tragbaren Brillengestells durch Probung verschiedener, dem Optiker zur Verfügung stehender Modelle hinsichtlich ihres Sitzes auf der Nase des betreffenden Klienten. Dabei ist Rücksicht darauf zu nehmen, daß die als Flügel ausgebildeten Anlageflächen des Brillengestells möglichst ganzflächig unter gleichmäßigem Druck anliegen, jedenfalls nicht einen punktuellen Druck auf die Nasenseitenflächen ausüben. Diese Verprobung erfordert beträchtliche Erfahrung seitens des Optikers, kann aber dennoch unter Umständen zeitraubend sein, besonders bei asymmetrischem Verlauf der Nasenflügel des Klienten zu seiner Augenstellung, oder bei anderweitigen Abweichungen seiner Nasenform.

In extremen Fällen kann eine Anpassung dadurch vorgenommen werden, daß zunächst ein Gipsmodell der Nase des Klienten — begrenzt auf den in Betracht kommenden Bereich — abgenommen und anhand dieses Gipsmodells die passende Brillenfassung angefertigt wird.

Wie leicht ersichtlich, sind alle diese vorbereitenden Maßnahmen aufwendig und zeitraubend, dabei aber dennoch mit Unsicherheitsfaktoren belastet. Durch die FR-A-2 267 736 ist ein Hilfsgerät für die Brillenanpassungstechnik bekannt geworden, das es gestattet, die Umrißlinie des Rückens und der Seitenflächen der Nase in einer für den vorgesehenen Zweck ausgewählten Ebene — vorzugsweise der Ebene des Nasenkeilwinkels — abzubilden. Das Gerät ist den bekannten Geräten zur Abtastung unregelmäßig gestalteter Oberflächen von Körpern entlang einer Umfangslinie mit Hilfe zahlreicher parallel nebeneinander in einer Führung angeordneter längsbeweglicher dünner stabförmiger Lamellen nachgebildet. Das Gerät erlaubt nicht die Feststellung bzw. Messung irgendwelcher Zahlenwerte, sondern nur eine konfigurative Nachbildung des Nasenrückens der betreffenden Person und die Auswertung des Ergebnisses dieser Nachbildung durch Anpassungsvorgänge.

Weiterhin ist ein Gerät bekannt (FR-A-2 497 655), mit dem die Nasenbreite unter Berücksichtigung vorgegebener Nasenwinkel meßbar ist. Dieses Gerät besteht aus zwei nach Art von Schiebern linear verschieblichen Bauteilen, die mehrere Ausschnitte mit schräg verlaufenden Anlageflächen für die Nasenflügel aufweisen. Jeder Ausschnitt weist verschieden schräge Anlageflächen auf, wobei die eine Anlagefläche an dem einen Schieber mit der gegenüberliegenden Anlagefläche des anderen Schiebers spiegelsymmetrisch ist. Es ist jedoch nur eine grobe Abschätzung, nicht aber eine Messung des Nasenwinkels möglich.

Der Erfindung liegt hingegen die Aufgabe zugrunde, die Bereitstellung von Meßwerten für verschiedene Nasenwinkel als Grundlage für die Anpassung einer Brille zu ermöglichen.

Ausgehend von dem eingangs genannten Gerät wird diese Aufgabe gemäß der Erfindung dadurch gelöst, daß die beiden Bauteile als um einen gemeinsamen Drehpunkt winkelbeweglich gegeneinander verschwenkbare Scheiben ausgebildet sind, von denen eine die linear verstellbare Anlagefläche aufweist, deren Verstellbarkeit unabhängig von der Winkellage der Anlageflächen zueinander ist, und eine Scheibe eine Winkel-Skala, die andere eine Meßmarke trägt, die sich in der Ausgangsposition des Geräts mit der Mittellinie der Winkelskala deckt.

Das Gerät wird nach erster Anpassung an die Nasenrückenbreite durch lineare Verschiebung der einen Anlagefläche gegenüber der anderen der Nase des Klienten aufgesetzt und die Winkelstellung der beiden Scheiben zueinander bis zur satten Anlage der Anlageflächen an den Außenflächen des oberen Teiles der Nase verändert; notwendigenfalls wird die Nasenrückenbreite nachträglich nochmals durch geringe Linearverschiebung, und der Nasenwinkel durch entsprechend geringe Veränderung der Winkelstellung der beiden Scheiben zueinander korrigiert. Mit Hilfe des so ermittelten Ergebnisses können die vorhandenen Brillengestelle schnell überprüft, notwendigenfalls kann ein genau passendes Gestell nach den gefundenen Meßwerten zur Anfertigung in Auftrag gegeben werden.

In zweckmäßiger Ausbildung besitzt mindestens eine der beiden Scheiben im Bereich ihrer Basislinie einen in einer Schlitzführung linear verschiebbaren Anpaßschieber, der mit seiner Unterseite die Basislinie der betreffenden Scheibe bildet und dessen eine Seitenkante die verstellbare Anlagefläche bildet. Für die Handhabung ist es günstig, wenn der Anpaßschieber in der Schlitzführung straff geführt ist, so daß er sich in der jeweils eingestellten Lage selbst fixiert. Durch die lineare Verschiebung des Anpaßschiebers wird die Winkelstellung der beiden Anlageflächen zueinander nicht verändert.

Eine bevorzugte Ausführungsform des erfindungsgemäßen Meßgerätes sieht vor, daß die beiden Scheiben kreissektorförmig mit gleichem Radius ausgebildet, durch einen gemeinsamen, den jeweiligen Kreismittelpunkt als Drehpunkt der beiden Scheiben durchdringenden Lagerzapfen drehbeweglich miteinander verbunden und mit je einem Basisteil versehen sind, deren Breite der Länge der Projektion der Anlageflächen auf ein Lot auf den Basislinien der Basisteile entspricht, wobei das eine Basisteil die Schlitzführung für den Anpaßschieber aufweist.

In bevorzugter Ausbildung dieser Ausführungsform ist die erste Scheibe annähernd halbkreisförmig, die zweite Scheibe annähernd viertelkreisförmig ausgebildet, wobei beide Scheiben im Bereich ihrer in der Ausgangsposition des Geräts fluchtenden Begrenzungsradien über diese hinaus zu den Basisteilen vergrößert sind. Dadurch wird erreicht, daß der Drehpunkt der beiden Anlageflächen so angeordnet werden kann, daß er sich bei der Handhabung unmittelbar über dem Nasenrücken befindet.

Im Falle der zuvor geschilderten Ausbildung mit halbkreisförmiger erster und viertelkreisförmiger zweiter Scheibe ist die Winkel-Skala zweckmäßig auf der ersten Scheibe in symmetrischer Anordnung zu deren Mittellinie angeordnet, und die Meßmarke der zweiten Scheibe fällt mit deren innerem Begrenzungsradius zusammen.

In zweckmäßiger weiterer Ausbildung der Erfindung besitzt die den Anpaßschieber tragende Scheibe in ihrem Basisteil eine metrische Skala zur Messung der Nasenrückenbreite, der auf dem Anpaßschieber eine Meßmarke zugeordnet ist. Damit ergibt sich die Möglichkeit, auch für die Nasenrückenbreite eine objektive Maßzahl festzustellen.

Schließlich erscheint es zweckmäßig, für beide Verstellmöglichkeiten, nämlich für die Winkelverschwenkung und für die lineare Verschiebung je eine Feststellvorrichtung, z. B. eine Feststellschraube vorzusehen, um die ermittelten Maße fixieren zu können.

Ein Ausführungsbeispiel der Erfindung ist in der anschließenden Beschreibung der beigegebenen Zeichnungen dargestellt.

In den Zeichnungen zeigen :

Figur 1 das gesamte Gerät in Ausgangsposition in Draufsicht ;

Figur 2 die erste Scheibe desselben mit Zubehörteilen, teilweise abgebrochen ;

Figur 3 die zweite Scheibe des Gerätes.

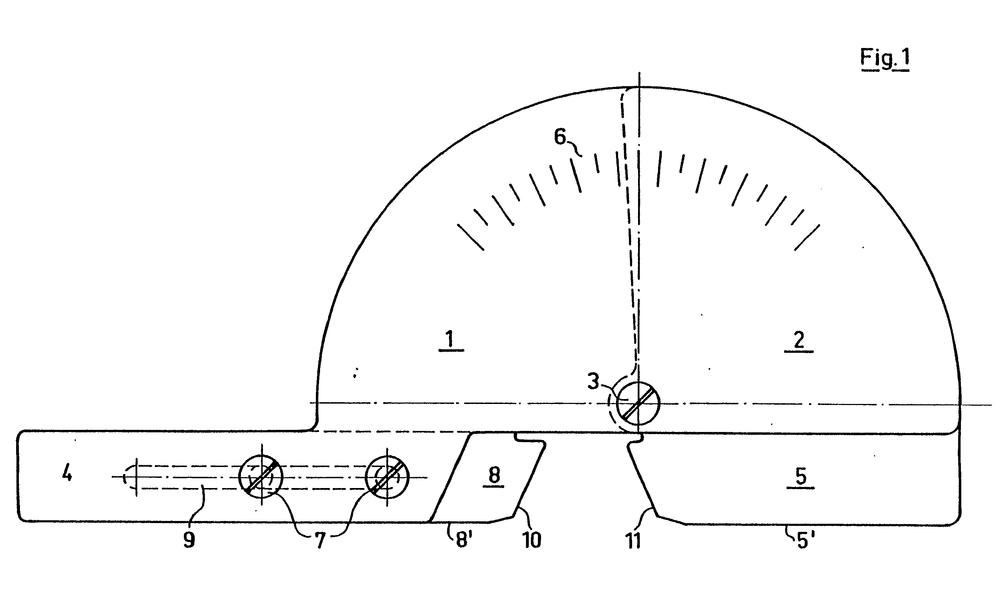

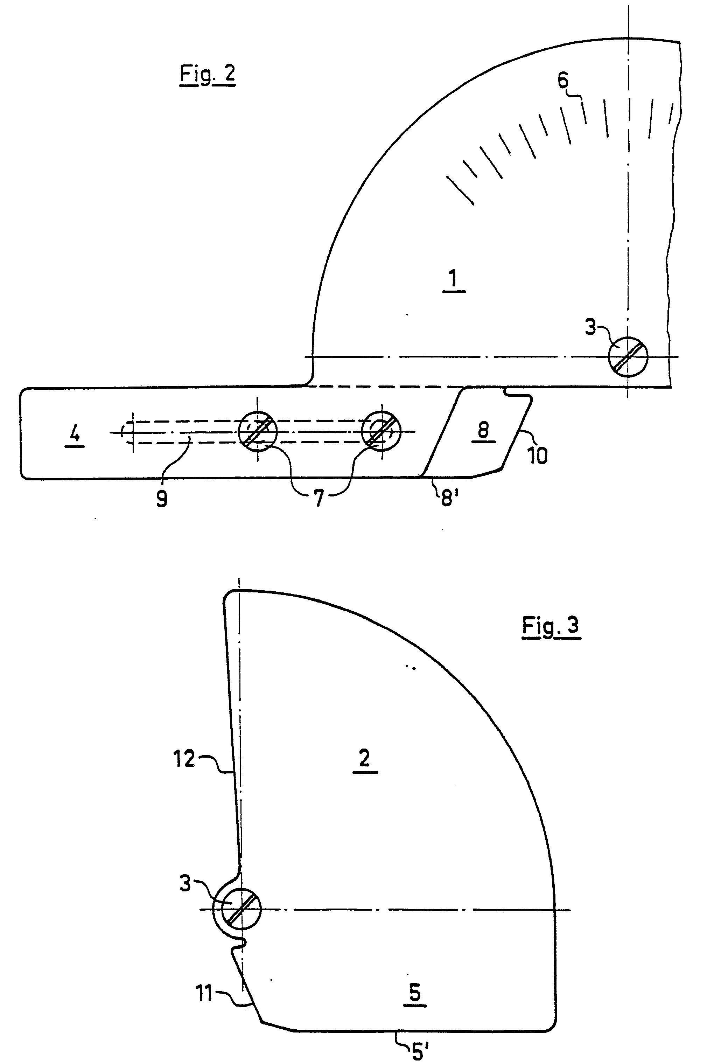

Das in den Figuren dargestellte Gerät ist in natürlicher Größe abgebildet. Es besteht aus einer ersten Scheibe 1 und einer zweiten Scheibe 2, die mittels eines eine Bohrung in beiden Scheiben durchdringenden Lagerzapfens 3 drehbeweglich miteinander verbunden (Figur 1) sind. Die Scheibe 1 ist im großen und ganzen halbkreisförmig ausgebildet, jedoch ist der Basisbereich ihrer einen — in der Zeichnung linken — Hälfte nach unten zu einem Basisteil 4 verbreitert ; in gleicher Weise besitzt die im großen und ganzen viertelkreisförmig ausgebildete Scheibe 2 einen um die gleiche Höhe nach unten auskragenden Basisteil 5 (Figur 3), der beim zusammengebauten Gerät zum Basisteil 4 der Scheibe 1 spiegelbildlich angeordnet ist. Die Scheibe 1 (in Figur 2 teilweise abgebrochen) trägt in ihrem oberen Teil eine Winkel-Skala 6 und an ihrem Basisteil 4 mittels zweier Führungsstifte 7 einen Anpaßschieber 8, der mittels einer Schlitzführung 9 und der Führungsstifte 7 gegen den Basisteil 4 linear verschiebbar ist.

Sämtliche flächigen Teile können aus beliebigem für solche Gegenstände geeignetem Material, z. B. Blech, Sperrholz, Kunststoff oder Glas bestehen ; geeignet ist auch eine Ausführungsform aus glasklarem Kunststoff in für die Handhabung ausreichender Stärke und Steifheit. Der Basisteil 5 der Scheibe 2 und der Anpaßschieber 8 besitzen je eine von ihren jeweiligen Basislinien 5', 8' aus ansteigende Schrägfläche, welche als Anlageflächen 10, 11 beide nach oben gegeneinander in bestimmtem Winkel konvergieren. Die vom Drehpunkt 3 aufsteigende vertikale Begrenzungslinie 12 der Scheibe 2 stellt die Meßmarke dieser Scheibe dar. Das Gerät wird wie folgt benutzt (s. Figur 1).

Das Gerät wird nach erster Anpassung der Nasenrückenbreite durch Horizontalverschiebung des Anpaßschiebers 8 der Nase des Klienten aufgesetzt und die Winkelstellung der beiden Scheiben 1, 2 zueinander bis zur satten Anlage der Anlagefläche 10, 11 an den Außenflächen des oberen Teils der Nase fixiert. Notwendigenfalls wird die Nasenrückenbreite nachträglich nochmals durch seitliche Verschiebung des Anpaßschiebers korrigiert. Der mittels der Begrenzungslinie 12 über der Winkel-Skala 6 abgelesene Winkel ist der zu messende Nasenkeilwinkel. Dabei ist allerdings zu beachten, daß die Skala in Ausgangsposition der Begrenzungslinie 12 bereits den Keilwinkel der beiden Anlageflächen in ihrer Ausgangslage zueinander anzeigen muß. Es verdient erwähnt zu werden, daß durch die Verschiebung des Anpaßschiebers 8 in Längsrichtung der Keilwinkel zwischen den Anlageflächen 10, 11 der beiden Teile 1, 2 nicht verändert wird ; jedoch wird im Gegensatz hierzu durch die Winkelverschwenkung der beiden Scheiben die zuvor eingestellte Nasenrückenbreite etwas verändert, so daß sich aus diesem Grunde die in der Handhabung erwähnt nachträgliche Korrektur als notwendig oder mindestens zweckmäßig erweist.

Die beiden Schrägflächen 10, 11 können senkrecht aus der Bildebene nach vorne und bzw. hinten herausragende dünne Flügel tragen, die eine breitere Anlage an den Nasenseitenflächen vermitteln, mittels deren dann aber beispielsweise auch die inneren Anlageflächen von Brillengestellen nachgemessen werden können.

Durch die Anbringung einer Skala an dem Anpaßschieber 8 und einer Meßmarke auf dem diesen tragenden Basisteil 4 (in der Zeichnung nicht dargestellt) ergibt sich die Möglichkeit, auch die Nasenrückenbreite messtechnisch zu erfassen. Ferner können sowohl für die Winkelverschwenkung, als auch für die Längsverschiebung Feststellschrauben vorgesehen sein, um die eingestellten Werte fixieren zu können.

## Patentansprüche

1. Gerät für die Brillenoptik zum Messen der Nasenwinkel unter Berücksichtigung der Nasenrückenbreite mit zwei flachen Bauteilen mit je einer schräg verlaufenden Anlagefläche für einen Nasenflügel, wobei die eine Anlagefläche gegenüber der anderen zur Einstellung auf die Nasenrückenbreite linear verstellbar ist und beide Anlageflächen in der Ausgangsposition des Gerätes spiegelsymmetrisch zu einer senkrecht zu der Bauteilebene und zu den Basislinien der Bauteile stehenden Ebene verlaufen, dadurch gekennzeichnet, daß die Bauteile als um einen gemeinsamen Drehpunkt (3) winkelbeweglich gegeneinander verschwenkbare Scheiben (1, 2) ausgebildet sind, von denen eine (1) die linear verstellbare Anlagefläche (10) aufweist, deren Verstellbarkeit unabhängig von der Winkellage der Anlageflächen (10, 11) zueinander ist, und eine Scheibe (1) eine Winkelskala (6), die andere (2) eine Meßmarke (12) trägt, die sich in der Ausgangsposition des Geräts mit der Mittellinie der Winkelskala (6) deckt.

2. Gerät nach Anspruch 1, dadurch gekennzeichnet, daß mindestens eine der beiden Scheiben (1) im Bereich ihrer Basislinie einen in einer Schlitzführung (9) linear verschiebbaren Anpaßschieber (8) besitzt, der mit seiner Unterseite die Basislinie (8') der Scheibe bildet und dessen eine Seitenkante die verstellbare Anlagefläche (10) bildet.

3. Gerät nach Anspruch 1 oder 2, dadurch gekennzeichnet, daß die beiden Scheiben (1, 2) kreissektorförmig mit gleichem Radius ausgebildet, durch einen gemeinsamen, den jeweiligen Kreismittelpunkt als Drehpunkt der beiden Scheiben durchdringenden Lagerzapfen (3) drehbeweglich miteinander verbunden und mit je einem Basisteil (4, 5) versehen sind, deren Breite der Länge der Projektion der Anlageflächen (10, 11) auf ein Lot auf den Basislinien der Basisteile entspricht, wobei das eine Basisteil (4) die Schlitzführung (9) für den Anpaßschieber (8) aufweist.

4. Gerät nach Anspruch 3, dadurch gekennzeichnet, daß die erste Scheibe (1) annähernd halbkreisförmig, die zweite Scheibe (2) annähernd viertelkreisförmig ausgebildet ist, wobei beide Scheiben im Bereich ihrer in der Ausgangsposition des Gerätes fluchtenden Begrenzungsradien über diese hinaus zu den Basisteilen (4, 5) vergrößert sind.

5. Gerät nach den Ansprüchen 1 und 4, dadurch gekennzeichnet, daß bei halbkreisförmiger Ausbildung der ersten und viertelkreisförmiger Ausbildung der zweiten Scheibe die Winkel-Skala (6) auf der ersten Scheibe in symmetrischer Anordnung zu deren Mittellinie angeordnet ist, und daß die Meßmarke der zweiten Scheibe (2) mit deren innerem Begrenzungsradius zusammenfällt.

6. Gerät nach einem der vorangehenden Ansprüche, dadurch gekennzeichnet, daß für die Winkelverschwenkung und für die lineare Verstellung je eine Feststellvorrichtung, z. B. eine Feststellschraube, vorgesehen ist.

7. Gerät nach Anspruch 3, dadurch gekennzeichnet, daß die erste Scheibe (1) im Basisteil (4) zur Messung der Nasenrückenbreite eine metrische Skala besitzt, der auf dem Anpaßschieber (8) eine Meßmarke zugeordnet ist.

## Claims

1. Apparatus for spectacle optics for measuring the nose angle, whilst taking account of the width of the bridge of the nose with two flat components with in each case one sloping contact surface for one side of the nose, one contact surface being linearly adjustable with respect to the other for adjusting to the width of the bridge of the nose and both contact surfaces in the initial position of the apparatus are mirror symmetrical to a plane perpendicular to the component plane and to the base lines of the components, characterized in that the components are constructed as disks (1, 2) pivotable with respect to one another and angularly movable about a common fulcrum (3), where one (1) has the linearly adjustable contact surface (10), whose adjustability is independent of the reciprocal angular position of the contact surfaces (10, 11), one disk (1) carrying an angular scale (6) and the other (2) a measuring mark (12) coinciding with the centre line of the angular scale (6) in the initial position of the apparatus.

2. Apparatus according to claim 1, characterized in that at least one of the two disks (1) has in the vicinity of its base line an adapting slide (8) linearly displaceable in a slot guide (9) and which with its underside forms the base line (8') of the disk and whereof one lateral edge forms the adjustable contact surface (10). ·

3. Apparatus according to claims 1 or 2, characterized in that the two disks (1, 2) are constructed in circular segment-like manner with the same radius, are connected in rotary manner to one another by a common pivot pin (3) passing through the particular centre of the circle as a fulcrum for the two disks and are provided with in each case one base part (4, 5), whose width corresponds to the length of the projection of the contact surfaces (10, 11) on a perpendicular line on the base line of the base parts, one base part (4) having the slot guide (9) for the adapting slide (8).

4. Apparatus according to claim 3, characterized in that the first disk (1) is approximately semicircular, the second disk (2) approximately quadrant-shaped and both disks are enlarged in the vicinity of their boundary radii which are aligned in the initial position of the apparatus and namely over and beyond the same to the base parts (4, 5).

5. Apparatus according to claims 1 and 4, characterized in that in the case of semicircular construction of the first and quadrant-shaped construction of the second disk, the angular scale (6) on the first disk is arranged symmetrically to the centre line thereof and that the measuring mark of the second disk (2) coincides with the inner boundary radius.

6. Apparatus according to one of the preceding claims, characterized in that in each case one fine setting, e. g. a setscrew is provided for the angular pivoting and for the linear adjustment.

7. Apparatus according to claim 3, characterized in that the first disk (1) has a metric scale for measuring the width of the bridge of the nose in base part (4) and with it is associated a measuring mark on the adapting slide (8).

**Revendications**

1. Appareil d'optique lunetière pour mesurer l'angle du nez en tenant compte de la largeur du dos du nez, comportant deux composants plats munis chacun d'une surface d'appui s'étendant en oblique pour une aile du nez, l'une des surfaces d'appui pouvant être réglée linéairement par rapport à l'autre pour se régler sur la largeur du dos du nez et les deux surfaces d'appui, dans la position de départ de l'appareil, s'étendant symétriques dans un miroir par rapport à un plan perpendiculaire au plan des composants et aux lignes de base des composants, caractérisé en ce que les composants sont réalisés en tant que disques (1, 2) pouvant basculer angulairement l'un par rapport à l'autre autour d'un point de rotation commun (3), dont l'un (1) comporte la surface d'appui réglable linéairement (10), dont le réglage est indépendant de la position angulaire des surfaces d'appui (10, 11) l'une par rapport à l'autre, et porte une graduation angulaire (6), tandis que l'autre (2) porte une marque de mesure (12) qui, dans la position de départ de l'appareil, coïncide avec la ligne médiane de la graduation angulaire (6).

2. Appareil selon la revendication 1, caractérisé en ce qu'au moins un des deux disques (1) comporte dans la zone de sa ligne de base un poussoir de réglage (8) pouvant se déplacer linéairement dans une fente de guidage (9), dont le côté inférieur constitue la ligne de base (8') du disque et dont l'une des arêtes latérales constitue la surface d'appui réglable (10).

3. Appareil selon la revendication 1 ou 2, caractérisé en ce que les deux disques (1, 2) sont réalisés sous forme de secteurs de cercle de même rayon, sont reliés ensemble à rotation par un tourillon commun (3) traversant en tant que point de rotation des deux disques le centre de chacun d'eux, et sont munis chacun d'une partie de base (4, 5) dont la largeur correspond à la longueur de la projection des surfaces d'appui (10) sur une perpendiculaire aux lignes de base des parties de base, l'une des parties de base (4) comportant la fente de guidage (9) pour le poussoir de réglage (8).

4. Appareil selon la revendication 3, caractérisé en ce que le premier disque (1) a sensiblement la forme d'un demi-cercle, le second disque (2) a sensiblement la forme d'un quart de cercle, les deux disques se prolongeant, dans la zone où leurs rayons de délimitation coïncident dans la position de départ de l'appareil, au-delà de ceux-ci en direction des lignes de base (4, 5).

5. Appareil selon les revendications 1 et 4, caractérisé en ce que, lorsque le premier disque a la forme d'un demi-cercle et le second disque la forme d'un quart de cercle, la graduation angulaire (6) est disposée sur le premier disque symétriquement par rapport à sa ligne médiane, et en ce que la marque de mesure du second disque (2) coïncide avec le rayon de délimitation intérieure de celui-ci.

6. Appareil selon l'une des revendications 1 et 4, caractérisé en ce qu'on prévoit pour le pivotement angulaire et pour le réglage linéaire respectivement un dispositif de blocage, par exemple une vis de blocage.

7. Appareil selon la revendication 3, caractérisé en ce que le premier disque (1) comporte dans la partie de base (4), pour mesurer la largeur du dos du nez une graduation métrique à laquelle est associée sur le poussoir de réglage (9) une marque de mesure.

Fig. 1

0 114 283

Fig. 2

Fig. 3

2